# EUROPEAN PATENT APPLICATION

(11) **EP 4 728 978 A1**
(43) Date of publication of application: **22.04.2026**
(21) Application number: 24846099.0
(22) Date of filing: 05.02.2024
(51) Int. Cl.: A61B 5/024, A61B 5/0245, A61B 5/00

(54) **METHOD AND ELECTRONIC DEVICE FOR MEASURING HEARTBEATS, AND STORAGE MEDIUM**

(30) Priority: 21.07.2023 KR 20230095510; 26.07.2023 KR 20230097487
(71) Applicant: Samsung Electronics Co., Ltd., Suwon-si, Gyeonggi-do 16677 (KR)
(72) Inventor: LEE, Jisoo, Suwon-si, Gyeonggi-do 16677 (KR); KIM, Hyogil, Suwon-si, Gyeonggi-do 16677 (KR)
(74) Representative: Gulde & Partner
(86) International application number: PCT/KR2024/095077
(87) International publication number: WO 2025/023798

(57) **Abstract**

An electronic device and a method for measuring heartbeats, and a storage medium are provided. The method for measuring heartbeats may comprise: determining whether there is valid ECG data measured within a preset time if an ECG measurement function is executed; determining whether a PPG measurement function is being executed in a wearable device if there is valid ECG data; and outputting a guide for guiding the execution of the PPG measurement function if the PPG measurement function is not being executed.

## Description

Embodiments of this document relate to an electronic device, a method, and a storage medium, for example, to an electronic device, a method, and a storage media for measuring heartbeats.

### [Background Art]

The disclosure relates to technology for analyzing the degree of irregularity in heartbeats using a PPG sensor in a wearable device, thereby alerting a user to the possibility of arrhythmia. There is technology for analyzing ECG sensor measurement values and detecting the presence of arrhythmia. However, unlike ECG signals that require a specific posture during measurement to measure cardiac action potentials, PPG signals offer the advantage of allowing unconscious continuous measurement and analysis as long as the user properly wears the wearable device.

Since the PPG sensor used in PPG-based irregular heartbeat detection technology is an optical-based sensor, the signal quality may vary significantly depending on the user's wearing condition and movement. In addition, unlike ECG signals, which directly measure cardiac action potentials, it may be an indirect monitoring method that observes changes in blood flow caused by heartbeats. Accordingly, detection accuracy is relatively low compared to ECG-based technologies, thereby potentially leading to user anxiety and unnecessary medical burden due to false detection.

The above-described information may be provided as related art to aid understanding of the disclosure. No claim or determination is made as to whether any of the above is applicable as prior art with respect to the disclosure.

### [Disclosure of Invention]

### [Solution to Problem]

A method for measuring heartbeats according to various embodiments of this document may determine whether there is valid ECG data measured within a pre-configured time when an ECG measurement function is executed. The method may determine whether a PPG measurement function is running in a wearable device when there is valid ECG data. The method may output a guide for execution of the PPG measurement function when the PPG function is not running.

An electronic device according to various embodiments of this document may include a display, a communication interface configured to communicate with a wearable device, and at least one processor. The at least one processor may determine whether there is valid ECG data measured within a pre-configured time when an ECG measurement function is executed. The at least one processor may determine whether a PPG measurement function is running in the wearable device when there is valid ECG data. The at least one processor may control the display to output a guide for execution of the PPG measurement function when the PPG function is not running.

A non-transitory computer-readable storage medium, recording a program for performing a method for measuring heartbeats according to various embodiments of this document, may perform determining whether there is valid ECG data measured within a pre-configured time when an ECG measurement function is executed. The storage medium may perform determining whether a PPG measurement function is running in a wearable device when there is valid ECG data. The storage medium may include outputting a guide for execution of the PPG measurement function when the PPG function is not running.

### [Advantageous Effects of Invention]

The electronic device, method, and storage medium for measuring heartbeats disclosed herein may complement the accuracy of PPG-based irregular heartbeat detection technology. Furthermore, the heartbeat measurement method and electronic device disclosed herein may guide the user to first become familiar with the ECG measurement method before using the relevant function, and when an irregular heartbeat detection alert occurs while using the function, the user may be guided to measure ECG to identify the result with higher accuracy.

### [Brief Description of Drawings]

FIG. 1 is a block diagram of an electronic device in a network environment according to various embodiments.
FIG. 2 is a block diagram illustrating the configuration of an electronic device according to various embodiments.
FIG. 3 is a block diagram illustrating the configuration of a wearable device according to various embodiments.
FIGS. 4A, 4B, and 5 are diagrams illustrating a wearable device according to various embodiments.
FIGS. 6A and 6B are flowcharts illustrating a heartbeat measurement method according to various embodiments.
FIG. 7 is a diagram illustrating a system including a single wearable device and an electronic device according to various embodiments.
FIGS. 8 and 9 are timing diagrams illustrating a system according to various embodiments.
FIG. 10 is a diagram illustrating a system including a plurality of wearable devices and an electronic device according to various embodiments.
FIGS. 11A, 11B, 11C, and 11D are diagrams illustrating a heartbeat measurement UI according to various embodiments.
FIG. 12 is a diagram illustrating a heartbeat measurement waveform according to various embodiments.
FIG. 13 is a diagram illustrating a UI that provides guidance on ECG measurement according to various embodiments.
FIG. 14 is a flowchart illustrating a heartbeat measurement method according to various embodiments.

### [Mode for the Invention]

Hereinafter, embodiments of the disclosure will be described in detail with reference to the drawings so that those skilled in the art to which the disclosure pertains are able to easily implement the disclosure. However, the disclosure may be implemented in various different forms and is not limited to the embodiments described herein. In connection with the description of the drawings, the same or similar reference numerals may be used for the same or similar components. In addition, in the drawings and related descriptions, descriptions of well-known functions and configurations may be omitted for clarity and conciseness.

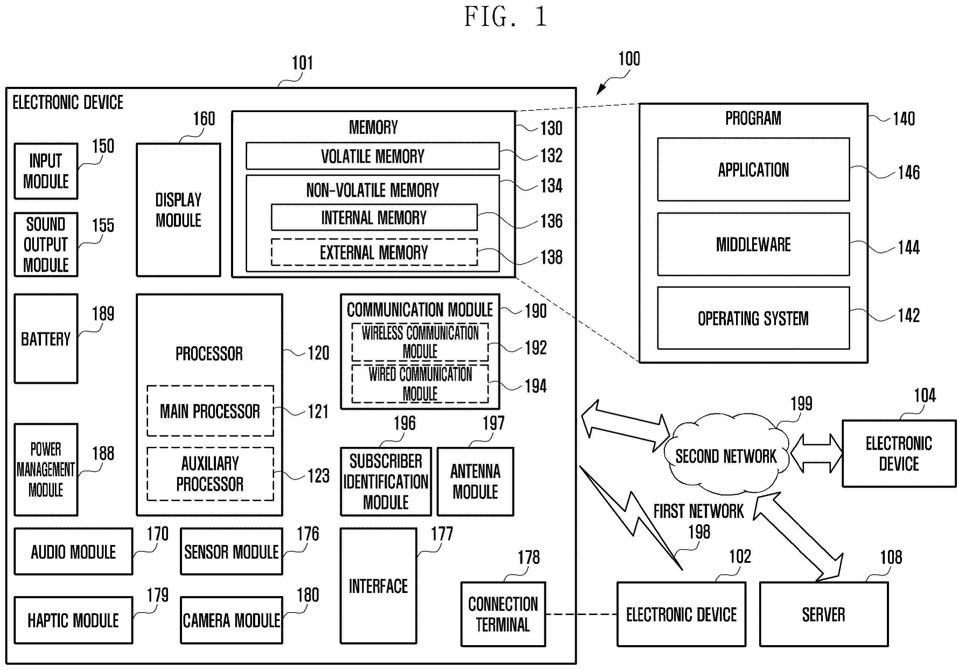

FIG. 1 is a block diagram illustrating an electronic device 101 in a network environment 100 according to various examples. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an example, the electronic device 101 may communicate with the electronic device 104 via the server 108. According to an example, the electronic device 101 may include a processor 120, memory 130, an input module 150, a sound output module 155, a display module 160, an audio module 170, a sensor module 176, an interface 177, a connection terminal 178, a haptic module 179, a camera module 180, a power management module 188, a battery 189, a communication module 190, a subscriber identification module (SIM) 196, or an antenna module 197. In some examples, at least one of the components (e.g., the connection terminal 178) may be omitted from the electronic device 101, or one or more other components may be added in the electronic device 101. In some examples, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one example, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an example, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an example, the auxiliary processor 123 (e.g., an image signal processor or a communication processor) may be implemented as part of another component (e.g., the camera module 180 or the communication module 190) functionally related to the auxiliary processor 123. According to an example, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134. The non-volatile memory may include at least one of an internal memory 136 and an external memory 138.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an example, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an example, the display module 160 may include a touch sensor adapted to detect a touch, or a pressure sensor adapted to measure the intensity of force incurred by the touch.

The audio module 170 may convert a sound into an electrical signal and vice versa. According to an example, the audio module 170 may obtain the sound via the input module 150, or output the sound via the sound output module 155 or a headphone of an external electronic device (e.g., the electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an example, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an example, the interface 177 may include, for example, a high definition multimedia interface (HDMI), a universal serial bus (USB) interface, a secure digital (SD) card interface, or an audio interface.

The connection terminal 178 may include a connector via which the electronic device 101 may be physically connected with the external electronic device (e.g., the electronic device 102). According to an example, the connection terminal 178 may include, for example, an HDMI connector, a USB connector, an SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an example, the haptic module 179 may include, for example, a motor, a piezoelectric element, or an electric stimulator.

The camera module 180 may capture a still image or moving images. According to an example, the camera module 180 may include one or more lenses, image sensors, image signal processors, or flashes.

The power management module 188 may manage power supplied to the electronic device 101. According to one example, the power management module 188 may be implemented as at least part of, for example, a power management integrated circuit (PMIC).

The battery 189 may supply power to at least one component of the electronic device 101. According to an example, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an example, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a fifth generation (5G) network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a fourth generation (4G) network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the millimeter wave (mmWave) band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an example, the wireless communication module 192 may support a peak data rate (e.g., 20 gigabits per second (Gbps) or more) for implementing eMBB, loss coverage (e.g., 164 decibels (dB) or less) for implementing mMTC, or U-plane latency (e.g., 0.5 milliseconds (ms) or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an example, the antenna module 197 may include an antenna including a radiating element composed of a conductive material or a conductive pattern formed in or on a substrate (e.g., a printed circuit board (PCB)). According to an example, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an example, another component (e.g., a radio frequency integrated circuit (RFIC)) other than the radiating element may be additionally formed as part of the antenna module 197.

According to various examples, the antenna module 197 may form an mmWave antenna module. According to an example, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an example, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an example, all or some of operations to be executed at the electronic device 101 may be executed at one or more of the external electronic devices (e.g. electronic devices 102 and 104 or the server 108). For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an example, the external electronic device 104 may include an internet-of-things (IoT) device. The server 108 may be an intelligent server using machine learning and/or a neural network. According to an example, the external electronic device 104 or the server 108 may be included in the second network 199. The electronic device 101 may be applied to intelligent services (e.g., smart home, smart city, smart car, or healthcare) based on 5G communication technology or IoT-related technology.

The electronic device according to various examples may be one of various types of electronic devices. The electronic devices may include, for example, a portable communication device (e.g., a smartphone), a computer device, a portable multimedia device, a portable medical device, a camera, a wearable device, or a home appliance. According to an example of the disclosure, the electronic devices are not limited to those described above.

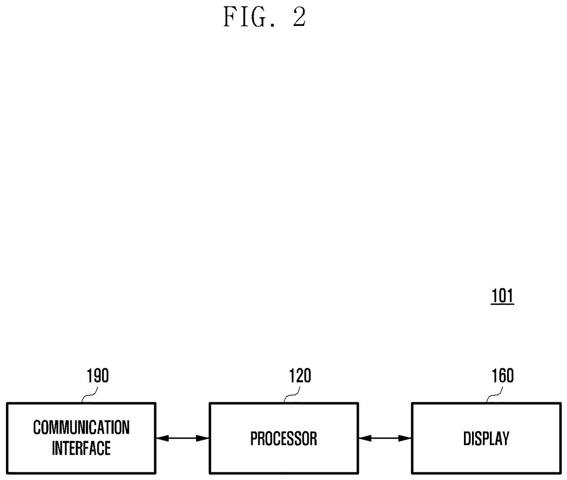

FIG. 2 is a block diagram illustrating the configuration of an electronic device according to various embodiments.

Referring to FIG. 2, an electronic device 101 (e.g., the electronic device 101 in FIG. 1) may include a communication interface 190, a processor 120, and a display 160. For example, the electronic device 101 may include a smartphone, a tablet PC, a desktop PC, a laptop PC, a smart TV, or a digital center fascia.

The communication interface 190 (e.g., the communication module 190 in FIG. 1) may communicate with a wearable device. For example, the communication interface 190 may transmit a heartbeat measurement command to the wearable device and receive heartbeat measurement data and/or analysis results from the wearable device. For example, the heartbeat measurement data may include photoplethysmography (PPG) data and electrocardiogram (ECG) data. The communication interface 190 may be referred to as a communication device, a communication unit, a communication module, a transmitting/receiving device, a transceiver, etc.

The display 160 (e.g., the display module 160 in FIG. 1) may display received heartbeat measurement data and/or analysis results. In addition, the display 160 may display a user interface (UI) (e.g., a guide UI) related to PPG measurement and/or ECG measurement.

The processor 120 (e.g., the processor 120 in FIG. 1) may control each component of the electronic device 101. The electronic device 101 may include one or more processors 120. When an ECG measurement function is executed, the processor 120 may determine whether there is valid ECG data measured within a pre-configured time. For example, the pre-configured time may be set to one week. For example, ECG data and/or ECG analysis results may include sinus rhythm, atrial fibrillation, analysis failure, and/or poor signal quality. When the ECG data and/or ECG analysis results include sinus rhythm, atrial fibrillation, and/or analysis failure, the wearable device 200 and/or the electronic device 101 may determine the ECG data as valid. Alternatively, when the ECG data and/or ECG analysis results include poor signal quality, the wearable device 200 and/or the electronic device 101 may determine the ECG data as invalid.

When there is no valid ECG data, the processor 120 may control the display 160 to output a UI for guiding ECG measurement. For example, when there is no measured ECG data, or the signal quality of the measured ECG data is poor, the processor 120 may determine that there is no valid ECG data. When there is no valid ECG data, the processor 120 may disable a PPG measurement function. In other words, the processor 120 may prompt the user to perform ECG measurement so that valid ECG data is obtained before PPG measurement. The processor 120 may control the communication interface 190 to transmit an ECG measurement command to the wearable device according to the user's command.

Alternatively, when there is valid ECG data, the processor 120 may determine whether the PPG measurement function is running on the wearable device. When the PPG function is running, the processor 120 may control the display 160 to display a UI indicating that the PPG function is running. Alternatively, when the PPG function is not running, the processor 120 may control the display 160 to output a UI for guiding the execution of the PPG measurement function.

The electronic device 101 may include a memory (e.g., the memory 130 in FIG. 1). The memory may store data, algorithms, and the like for executing the functions of the electronic device 101, and may store programs, instructions, and the like executed on the electronic device 101. For example, the memory may include a UI module, an ECG analysis history identification module, an ECG analysis module, an ECG validity analysis identification module, a PPG analysis module, and/or a storage module.

The UI module may display buttons for user input, measurement guides, and/or heartbeat measurement data on the display 160. The ECG analysis history identification module may receive events from the UI module, identifies whether past user ECG sensor measurements and analysis history exist, and control the ECG validity analysis identification module for the most recent analysis. The ECG analysis module may receive events from the UI module and analyze measured ECG data. The ECG validity analysis identification module may receive analysis results from the measurement history identification module and/or the analysis module to identify whether the analysis is valid. The PPG analysis module may receive events from the ECG analysis module and analyze measured PPG data. For example, when PPG data is stored in the memory, the PPG analysis module may detect a peak value of a biometric signal from the PPG data and calculate heartbeats, based on the peak-to-peak interval. The PPG analysis module may calculate stress, based on the variance of the peak-to-peak interval and/or data converted to the frequency domain. The storage module may store ECG analysis results (and/or ECG data) and/or PPG analysis results (and/or PPG data). Data, algorithms, programs, and/or instructions stored in the memory may be loaded into the processor 120 and executed thereon.

The wearable device may continuously execute the PPG measurement function. When the PPG measurement function is executed in the wearable device, the processor 120 may control the communication interface 190 to receive PPG data from the wearable device at a pre-configured cycle. For example, the processor 120 may receive PPG data from the wearable device at regular time intervals. Alternatively, the processor 120 may receive PPG data from the wearable device whenever the wearable device performs a measurement.

When the wearable device executes the PPG measurement function, the processor 120 may transmit an irregular heartbeat monitoring function execution command to the wearable device according to a user command. The wearable device may monitor whether the PPG data includes irregular heartbeat information, based on the received irregular heartbeat monitoring function execution command. When the PPG data received from the wearable device includes irregular heartbeat information a pre-configured number of times or more, the display 160 may be controlled to output a UI for guiding ECG measurement. For example, when the received PPG data includes irregular heartbeat information even once, the processor 120 may control the display 160 to output a UI for guiding ECG measurement. Alternatively, when the received PPG data includes irregular heartbeat information three or more times, the processor 120 may control the display 160 to output a UI for guiding ECG measurement. In addition, when the measured ECG data includes irregular heartbeat information, the electronic device 101 may transmit the ECG data (and/or analysis results), the PPG data (and/or analysis results), and/or a medical service request to a server of an external organization (e.g., a hospital).

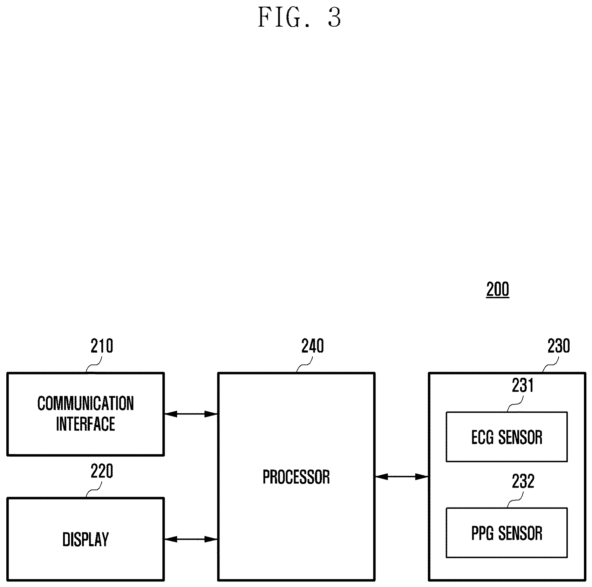

FIG. 3 is a block diagram illustrating the configuration of a wearable device according to various embodiments.

Referring to FIG. 3, a wearable device 200 (e.g., the electronic device 101 in FIG. 1) may include a communication interface 210, a display 220, a sensor 230, and a processor 240. For example, the wearable device may include a smartwatch and a smart ring (e.g., a smart bracelet, a smart band, or a smart anklet).

The communication interface 210 may communicate with the electronic device 101. For example, the communication interface 210 may receive a heartbeat measurement command from the electronic device 101 and transmit heartbeat measurement data (e.g., ECG data and PPG data) and/or analysis results to the electronic device 101. The communication interface 210 may also be referred to as a communication device, a communication unit, a communication module, a transmitting/receiving device, a transceiver, etc.

The display 220 (e.g., the display module 160 in FIG. 1) may display measured heartbeat data and/or analysis results. In addition, the display 220 may display a user interface (UI) related to PPG measurement and/or ECG measurement.

The sensor 230 (e.g., the sensor module 176 in FIG. 1) may include an ECG sensor 231 and a PPG sensor 232. The ECG sensor 231 may include electrodes and measure ECG when a user touches the electrodes and executes a command. The PPG sensor 232 may include an emitter and a receiver. The emitter may irradiate light onto the user's blood vessels, and the receiver may measure PPG by receiving light reflected from the blood vessels.

The processor 240 (the processor 120 in FIG. 1) may control each component of the electronic device 101. The wearable device 200 may include one or more processors 240. The processor 240 may control the ECG sensor 231 to measure ECG or the PPG sensor 232 to measure PPG, based on a heartbeat measurement command received through the communication interface 210. Alternatively, the wearable device 200 may include an input interface and receive a heartbeat measurement command from the user through the input interface. The processor 240 may execute a heartbeat measurement function by controlling the ECG sensor 231 or the PPG sensor 232, based on the input user measurement command.

The processor 240 may control the display 220 to display a UI related to heartbeat measurement. For example, when there is no valid ECG data, the processor 240 may display a UI for guiding ECG measurement through the display 220. In other words, the processor 240 may guide the user to use the irregular heartbeat alert function using the PPG sensor 232 after obtaining valid analysis results using the ECG sensor 231. When irregular heartbeat data is included in the PPG data, the processor 240 may display a UI for guiding the user to measure the ECG via the display 220. In addition, the processor 240 may display a UI indicating the start of heartbeat measurement, a UI guiding a heartbeat measurement method, a UI indicating that heartbeat measurement is in progress, and/or a UI indicating the end of heartbeat measurement via the display 220. Alternatively, the processor 240 may display heartbeat measurement data and/or analysis results via the display 160.

The wearable device 200 may include a memory (e.g., the memory 130 in FIG. 1). The memory may store data, algorithms, and the like for executing the functions of the wearable device 200, and may store programs, instructions, and the like executed on the wearable device 200. For example, the memory may include a UI module, an ECG analysis history identification module, an ECG analysis module, an ECG sensor module, an ECG validity analysis identification module, a PPG analysis module, a PPG sensor module, and/or a storage module.

The UI module may display buttons for user input, measurement guides, and/or heartbeat measurement data on the display 220. The ECG analysis history identification module may receive events from the UI module, identifies whether past user ECG sensor measurements and analysis history exist, and control the ECG validity analysis identification module for the most recent analysis. The ECG analysis module may receive events from the UI module, control the ECG sensor module, and analyze ECG data measured from the ECG sensor. The ECG sensor module may control ECG sensor hardware. The ECG validity analysis identification module may receive analysis results from the measurement history identification module, measurement module, and/or analysis module to identify the validity of the analysis. The PPG analysis module may receive events from the ECG analysis module, control the PPG sensor module, and analyze PPG data measured from the PPG sensor. For example, when PPG data is stored in the memory, the PPG analysis module may detect a peak value of a biometric signal from the PPG data and calculate heartbeats, based on the peak-to-peak interval. The PPG analysis module may calculate stress, based on the variance of the peak-to-peak interval and/or data converted to the frequency domain. The PPG sensor module may control PPG sensor hardware. For example, the PPG sensor module may also monitor irregular heartbeats by modeling the frequency, intensity, and/or peak interval irregularities of the detected peak values. The storage module may store ECG analysis results (and/or ECG data) and/or PPG analysis results (and/or PPG data). Data, algorithms, programs, and/or instructions stored in the memory may be loaded into the processor 240 and executed thereon.

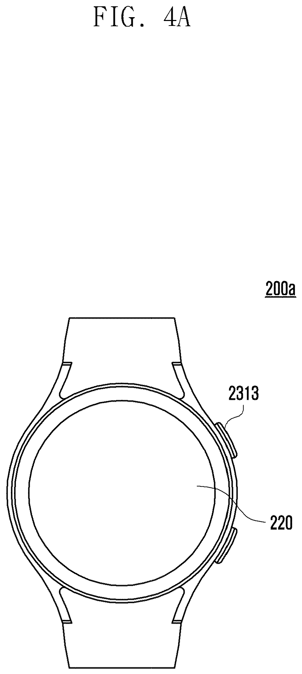

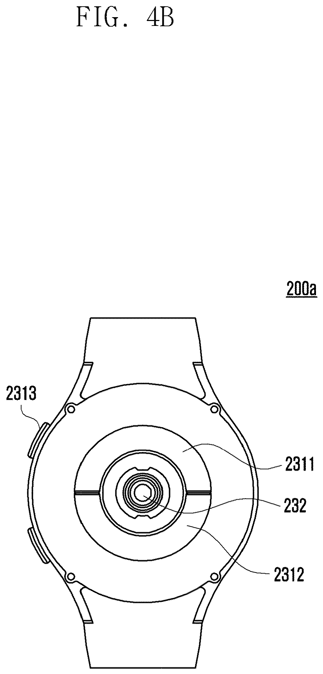

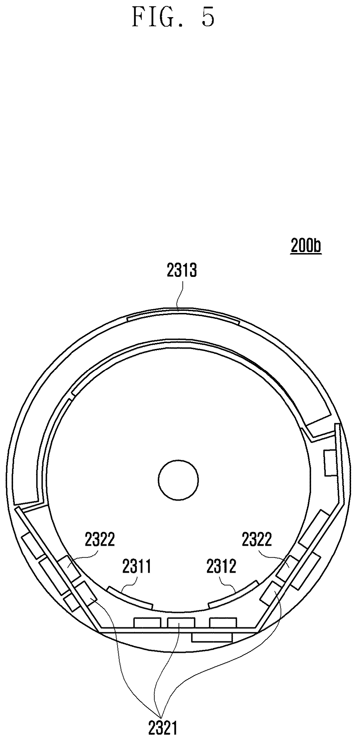

FIGS. 4A, 4B, and 5 are diagrams illustrating a wearable device according to various embodiments.

Referring to FIGS. 4A and 4B, a smartwatch 200a is illustrated as an example of a wearable device 200. The front face of the smartwatch 200a may include a display 220. For example, the display 220 may be implemented as a touchscreen and may receive user commands through the display 220.

A PPG sensor 232 may be disposed on a region (e.g., the central region) of the rear face of the smartwatch 200a. In addition, a first electrode 2311 and a second electrode 2322 of an ECG sensor 231 may be disposed on the rear face of the smartwatch 200a. Furthermore, a third electrode 2313 of the ECG sensor 231 may be disposed on the lateral face of the smartwatch 200a.

For example, the ECG sensor 231 may include a plurality of electrode interfaces (e.g., a first electrode, a second electrode, and a third electrode) and a sensor (e.g., an analog front end or a sensor processor) that processes collected biometric signals. The ECG sensor 231 may include two or more electrodes. For example, as illustrated in FIG. 4B, the ECG sensor 231 may include a first electrode 2311 that contacts the portion (e.g., the left wrist) where the smartwatch 200a is worn, a third electrode 2313 that contacts the portion (e.g., the right finger) opposite the wearing portion, and a second electrode 2312 that forms a bio-ground.

For example, the PPG sensor 232 may include an emitter and a receiver. For example, the emitter may include a light-emitting diode (LED) and/or a laser diode (LD). Light of a specific band emitted from the LED and/or LD may be absorbed, scattered, or reflected by the body, and the processor 240 may monitor changes in the light received by the receiver to identify physiological characteristics. For example, irregular heart rhythm notification (IHRN) is part of the heartbeat monitoring process, and the processor 240 may determine an irregular pattern among the acquired heartbeat information and notify the user or transmit the irregular heartbeat information to the electronic device 101.

Referring to FIG. 5, a smart ring 200b is illustrated as an example of a wearable device 200. For example, the smart ring 200b may include a smart band, a smart bracelet, or a smart anklet.

The smart ring 200b may include an ECG sensor 231 and a PPG sensor 232. The ECG sensor 231 may include a plurality of electrodes. As an example, a first electrode 2311 that contacts the portion (e.g., the left finger) where the smart ring 200b is worn and a second electrode 2312 that forms a bio-ground may be disposed on the inner ring of the smart ring 200b, while a third electrode 2313 that contacts the portion (e.g., the right finger) opposite the wearing portion may be disposed on the outer ring. In addition, the PPG sensor 232 may include an emitter 2321 and a receiver 2322. The emitter 2321 and receiver 2322 may be disposed on the inner ring of the smart ring 200b.

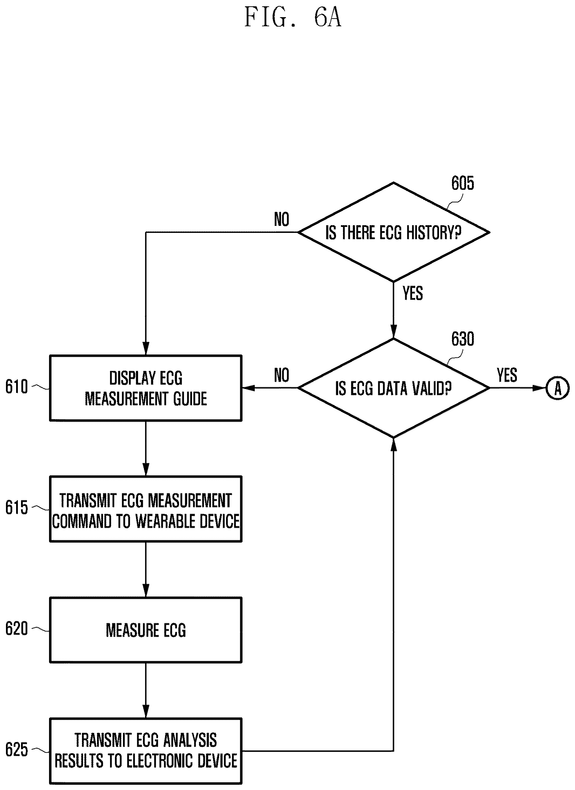

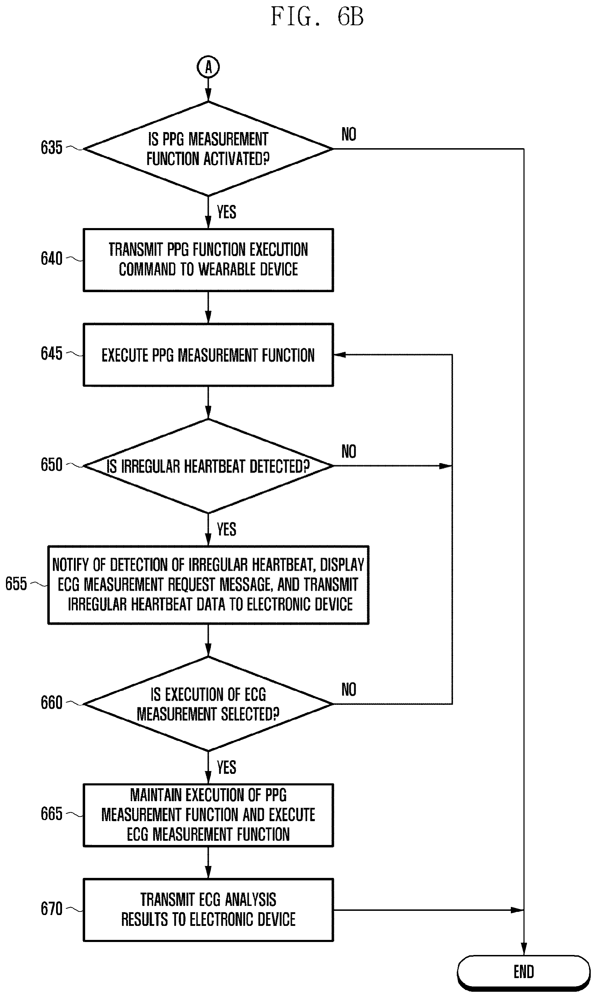

FIGS. 6A and 6B are flowcharts illustrating a heartbeat measurement method according to various embodiments.

In the following embodiments, respective operations may be performed sequentially, but are not necessarily performed sequentially. For example, the sequence of the respective operations may vary, and at least two operations may be performed in parallel.

According to an embodiment, operations 605 to 670 may be understood to be performed by a processor (e.g., the processor 120 in FIG. 2) of an electronic device 101 (e.g., electronic device 101 in FIG. 2) and/or a wearable device 200 (e.g., the wearable device 200 in FIG. 3).

The heartbeat measurement method and electronic device of this document may provide guidance on an ECG measurement method before the user uses a PPG-based irregular heartbeat detection function, and may help the user identify more accurate results using an ECG sensor when an alert occurs during use of the function.

Referring to FIG. 6A, an ECG measurement process is illustrated.

The electronic device 101 may display a UI for the user to execute an ECG function. When a heartbeat measurement command is input by the user, the electronic device 101 may determine whether there is an ECG measurement history (605). When there is an ECG measurement history (Yes in 605), the electronic device 101 may determine whether the ECG data is valid (630). When the ECG data is valid (Yes in 630), the electronic device 101 may perform a PPG measurement process (630-a). When there is no ECG history (No in 605) or the ECG data is invalid (No in 630, the electronic device 101 may display an ECG measurement guide (610). For example, the ECG measurement guide may be a UI that provides guidance on the ECG measurement method and/or measurement process to the user. The electronic device 101 may transmit an ECG measurement command to the wearable device 200 (615).

The wearable device 200 may receive the ECG measurement command from the electronic device 101 and display a UI for ECG measurement. In addition, the wearable device 200 may measure the ECG (620). When the ECG measurement is complete, the wearable device 200 may display the measured ECG data and/or analysis results. In addition, the wearable device 200 may transmit the ECG analysis results to the electronic device 101 (625).

The electronic device 101 may determine whether the received ECG data (or analysis results) is valid (630). For example, when the signal quality of the ECG data is poor, the electronic device 101 may determine the ECG data as invalid. When the ECG data is invalid (No in 630), the electronic device 101 may display an ECG measurement guide (610). The electronic device 101 may display a UI for ECG measurement and guide the user to re-measure the ECG through the wearable device 200. When there is no valid ECG data, the electronic device 101 may restrict (or prohibit or disable) the PPG measurement function. When the ECG data is valid (Yes in 630), the electronic device 101 may perform the PPG measurement process (630-a).

Referring to FIG. 6B, the PPG measurement process is illustrated.

The electronic device 101 may determine whether the PPG measurement function is activated (635). For example, the PPG sensor 232 may continuously measure irregular heartbeat information (IHRN). The PPG measurement function may include an alert function for detecting irregular heartbeat information. When the PPG measurement function is not activated (No in 635), the electronic device 101 may display a UI indicating the deactivation state of the PPG measurement function and inquiring whether to activate it. For example, the UI inquiring whether to activate it may include a toggle key for selecting monitoring of irregular heartbeat information. When the toggle key is toggled to (or maintained in) a deactivated state, the electronic device 101 and/or the wearable device 200 may not perform monitoring of irregular heartbeat information. When the toggle key is toggled to (or maintained in) an activated state, the electronic device 101 may activate the monitoring function for irregular heartbeat information. When the PPG measurement function is activated (Yes in 635), the electronic device 101 may display a UI indicating that the PPG measurement function is activated. In addition, the electronic device 101 may transmit a PPG function execution command to the wearable device 200 (640). Additionally, when the monitoring function for irregular heartbeat information is activated, a command to execute monitoring of irregular heartbeat information may be transmitted.

When the wearable device 200 receives a PPG function execution command and/or a command to execute monitoring of irregular heartbeat information, the wearable device 200 may execute a PPG measurement function and/or irregular heartbeat information monitoring function (645). The wearable device 200 may continuously perform the PPG measurement function and detect irregular heartbeat information. When irregular heartbeat information is not detected (No in 650), the wearable device 200 may continuously perform the PPG measurement function (645). The wearable device 200 may transmit PPG data to the electronic device 101 at regular cycles or transmit PPG data to the electronic device 101 when an event occurs. When irregular heartbeat information is detected (Yes in 650), the wearable device 200 may notify the user of the detection of irregular heartbeat information and display a guide for ECG measurement (655). In addition, the wearable device 200 may transmit the detected irregular heartbeat information to the electronic device 101

(655). The electronic device 101 may store the received irregular heartbeat information. Alternatively, when the wearable device 200 detects irregular heartbeat information, it may transmit the detected irregular heartbeat information, along with an irregular heartbeat information detection event, to the electronic device 101. When the electronic device 101 receives the event, the electronic device 101 may notify the user of the detection of irregular heartbeat information and display a UI for guiding ECG measurement (655). The wearable device 200 may generate an event when PPG data includes irregular heartbeat information (or when irregular heartbeat information is detected). Alternatively, the wearable device 200 may generate an event when the PPG data includes irregular heartbeat information a pre-configured number of times or more.

The wearable device 200 and/or the electronic device 101 may determine whether the user executes ECG measurement (660). When execution of ECG measurement is not selected (No in 660), the wearable device 200 may continue to perform the PPG measurement function (645). When execution of ECG measurement is selected (Yes in 660), the wearable device 200 may display an ECG measurement UI and execute the ECG measurement function (665). At this time, the wearable device 200 may continue to execute the PPG measurement function. The wearable device 200 may analyze measured ECG data and display the ECG data and/or analysis results. In addition, the wearable device 200 may transmit the measured ECG data and/or the analysis results to the electronic device 101 (670).

The electronic device 101 may store the received ECG data and/or analysis results and display the ECG data and/or analysis results. In addition, the electronic device 101 may transmit the ECG data, the PPG data, and/or a medical service request to a server of an external organization (e.g., a hospital).

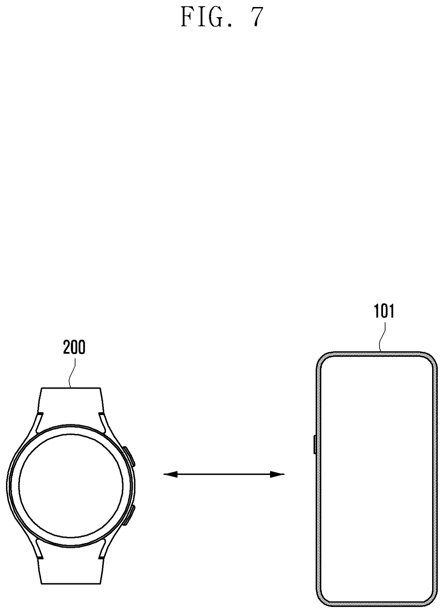

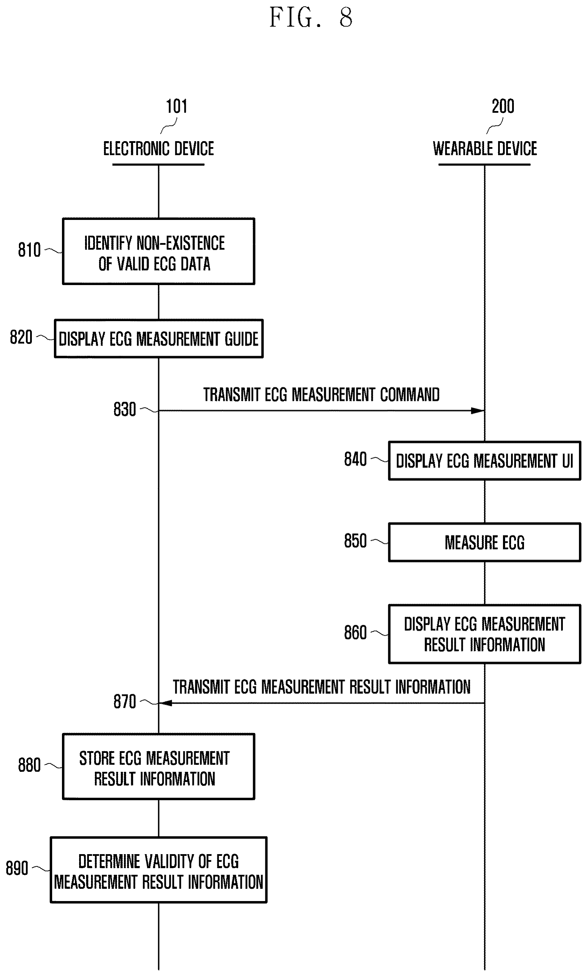

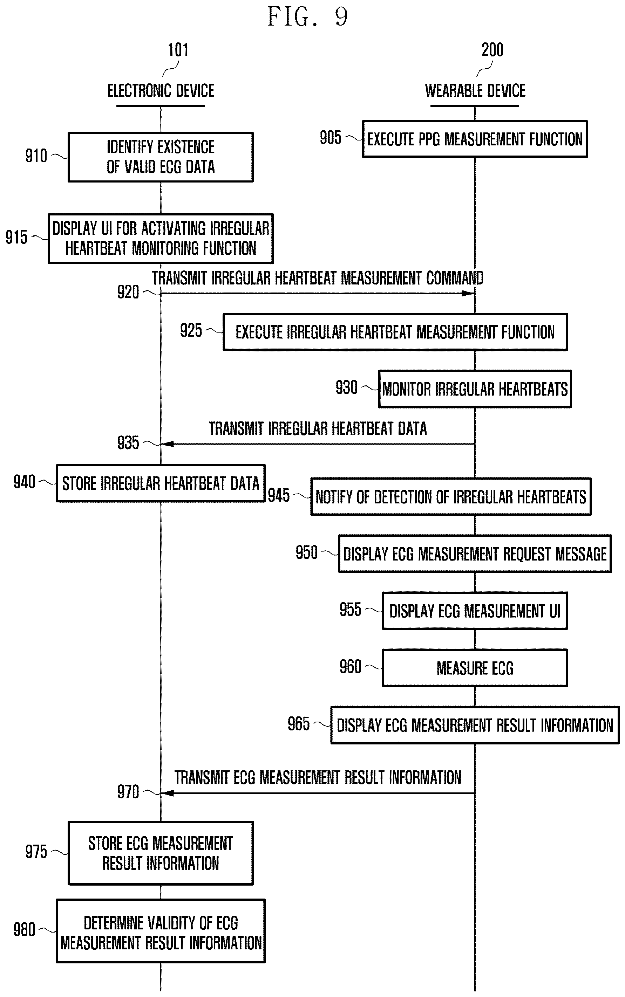

FIG. 7 is a diagram illustrating a system including a single wearable device and an electronic device according to various embodiments, and FIGS. 8 and 9 are timing diagrams illustrating a system according to various embodiments.

In the following embodiments, respective operations may be performed sequentially, but are not necessarily performed sequentially. For example, the sequence of the respective operations may vary, and at least two operations may be performed in parallel.

As an example, as illustrated in FIG. 7, the heartbeat measurement method of this document may be performed between an electronic device 101 and a single wearable device 200. The electronic device 101 may determine whether there is an ECG measurement history or whether ECG data is valid.

Referring to FIG. 8, when there is no ECG measurement history or the ECG data is invalid, the electronic device 101 may determine that valid ECG data does not exist (810). The electronic device 101 may display a guide for ECG measurement to the user (820). When an ECG measurement command is input from the user, the electronic device 101 may transmit an ECG measurement command to the wearable device 200 (830). When the wearable device 200 receives the ECG measurement command from the electronic device 101, the wearable device 200 may display a UI for ECG measurement (840). For example, ECG measurement may be performed by the ECG sensor 231. The ECG sensor 231 may include a plurality of electrodes and may measure ECG through the user's body in contact with the plurality of electrodes. When the ECG measurement is possible, the wearable device 200 may measure ECG (850). When the ECG measurement is complete, the wearable device 200 may display measurement result information (860). For example, the measurement result information may include measured ECG data. Alternatively, the wearable device 200 may analyze the measured ECG data and display analysis results. The wearable device 200 may transmit the ECG measurement result information to the electronic device 101 (870).

The electronic device 101 may store the received ECG measurement result information (880). Additionally, the electronic device 101 may determine the validity of the ECG measurement result information (890). For example, when the signal quality of the ECG measurement data is poor, the electronic device 101 may determine that the ECG measurement data is invalid. In this case, the electronic device 101 may guide the user to re-measure the ECG.

Referring to FIG. 9, the wearable device 200 may be executing a PPG measurement function (905). When there is an ECG measurement history and the ECG data is valid, the electronic device 101 may determine that valid ECG data exists (910). The electronic device 101 may display a UI for activating irregular heartbeat monitoring function (915). For example, the electronic device 101 may display a UI including a toggle key for selecting activation of the irregular heartbeat monitoring function. When the toggle key is moved to the activation state of the irregular heartbeat monitoring function, the electronic device 101 may transmit an execution command for the irregular heartbeat monitoring function to the wearable device 200 (920). The wearable device 200 may receive the execution command for the irregular heartbeat monitoring function and execute an irregular heartbeat detection function (925). The wearable device 200 may monitor irregular heartbeats (930). The wearable device 200 may continuously (or periodically) execute the PPG measurement function and detect irregular heartbeat information. When irregular heartbeat information is detected, the wearable device 200 may transmit the detected irregular heartbeat data to the electronic device 101 (935). The electronic device 101 may store the irregular heartbeat data (940).

In addition, the wearable device 200 may output an irregular heartbeat detection alert (945) and display an ECG measurement request message (950). For example, the ECG measurement request message may include a message recommending and/or guiding the user to perform ECG measurement. When the user selects ECG measurement, the wearable device 200 may display an ECG measurement UI (955). When ECG measurement is possible, the wearable device 200 may measure ECG (960). When the ECG measurement is completed, the wearable device 200 may display measurement result information (965). For example, the measurement result information may include measured ECG data. Alternatively, the wearable device 200 may analyze the measured ECG data and display analysis results. The wearable device 200 may transmit the ECG measurement result information to the electronic device 101 (970).

The electronic device 101 may store the received ECG measurement result information (975). In addition, the electronic device 101 may determine the validity of the ECG measurement result information (980). For example, when the signal quality of the ECG measurement data is poor, the electronic device 101 may determine the ECG measurement data to be invalid. In this case, the electronic device 101 may guide the user to re-measure the ECG. When irregular heartbeat information is included in the ECG data, the electronic device 101 may transmit the ECG data, the PPG data, and/or a medical service request to a server of an external organization (e.g., a hospital).

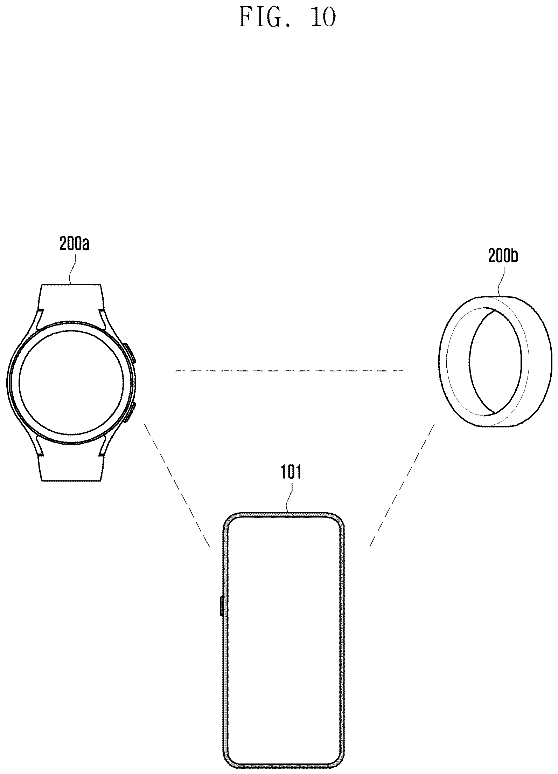

FIG. 10 is a diagram illustrating a system including a plurality of wearable devices and an electronic device according to various embodiments.

Referring to FIG. 10, a smartwatch 200a, a smart ring 200b, and an electronic device 101 (e.g., a smartphone) are illustrated. The heartbeat measurement method may be implemented in a system including one electronic device 100 and one wearable device 200, as illustrated in FIG. 7, or in a system including one electronic device 100 and a plurality of wearable devices 200a and 200b, as illustrated in FIG. 10.

For example, a smartwatch 200a and a smart ring 200b, which are wearable devices 200, may be in communication with an electronic device 101. The electronic device 100 may serve as a hub (e.g., operations 810 to 830, 880, and 890 in FIG. 8, and operations 910, 915, 940, 975, and 980 in FIG. 9 ), and the smartwatch 200a and the smart ring 200b may measure heartbeat information (e.g., ECG, PPG) (e.g., operations 840 to 870 in FIG. 8, and operations 905 and 925 to 965 in FIG. 9 ). Irregular heartbeat information included in the heartbeat information may be displayed on the electronic device 101.

In addition to the examples described above, the heartbeat measurement method may be implemented using a combination of various types of electronic devices.

For example, the electronic device 101 may collect (receive), analyze, and/or display heartbeat information, and the wearable device 200 (e.g., a smartwatch or smart ring) may measure heartbeat information. Alternatively, the smartwatch 200a (or smart ring 200b) may collect (receive), analyze, and/or display heartbeat information, and the smart ring 200b (or smartwatch 200a) may measure the heartbeat information. In this case, the smartwatch 200a may display a UI (e.g., a UI for identifying whether heartbeats are measured, a guide UI for a method or process, etc.) related to heartbeat measurement, thereby guiding the user to measure the heartbeat information. Measured irregular heartbeat information may be received from the smart ring 200b and an alert may be provided to the user.

Alternatively, the smartwatch 200a (or smart ring 200b) may collect (receive), analyze, and display heartbeat information and/or measure ECG, and the smart ring 200b (or smartwatch 200a) may determine irregular heartbeat information detected during the PPG measurement process. In this case, the smartwatch 200a may display a UI related to heartbeat measurement to guide the user to perform ECG measurement on the smartwatch 200a. In addition, the smartwatch 200a may guide the user to perform PPG measurement on the smart ring 200b.

Alternatively, the smartwatch 200a (or the smart ring 200b) may collect (receive), analyze, and display heartbeat information and/or measure ECG, and the smart ring 200b (or the smartwatch 200a) may determine irregular heartbeat information detected during the PPG measurement process. Alternatively, the electronic device 101 may collect (receive), analyze, and/or display heartbeat information, and the smartwatch 200a (or the smart ring 200b) may measure ECG, and the smart ring 200b (or the smartwatch 200a) may determine irregular heartbeat information detected during the PPG measurement process. In this case, irregular heartbeat information detected by the smart ring 200b may be transmitted to the electronic device 101, and the electronic device 101 may display the received irregular heartbeat information and display a UI for guiding the user to measure the ECG through the smartwatch 200. In addition, the electronic device 101 may display a UI guiding the user to seek medical consultation, based on the ECG data and/or analysis results received from the smartwatch 200, and request medical consultation and/or treatment services from a medical institution.

Alternatively, the above-described heartbeat measurement method may be performed solely by one wearable device 200 (e.g., the smartwatch 200a or the smart ring 200b). In this case, all of the above-described processes may be performed by one wearable device 200.

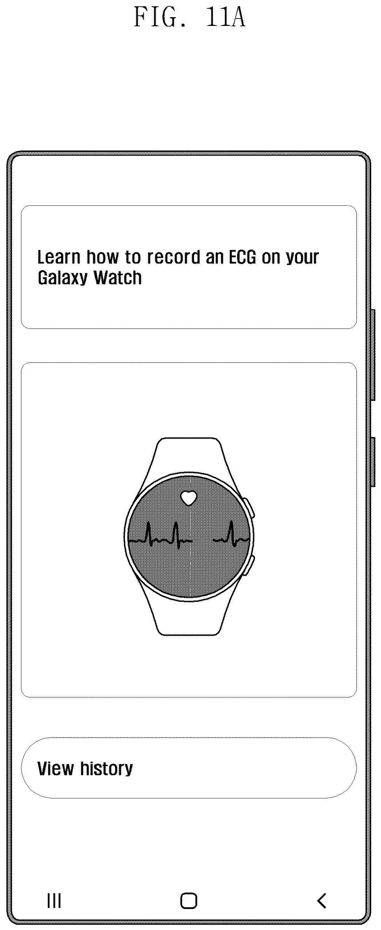

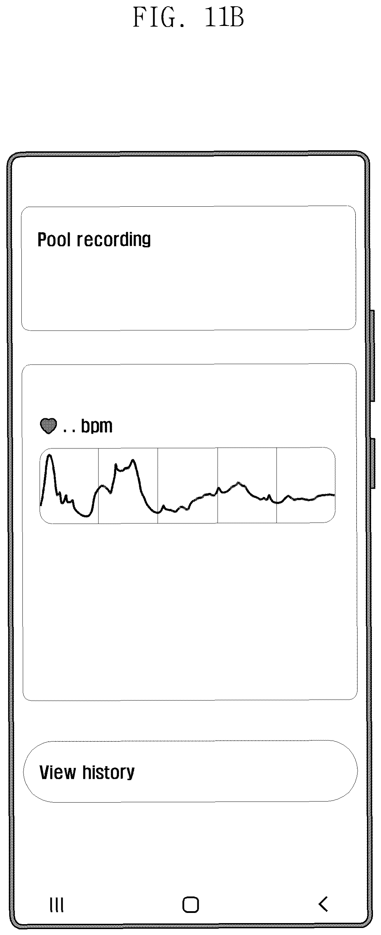

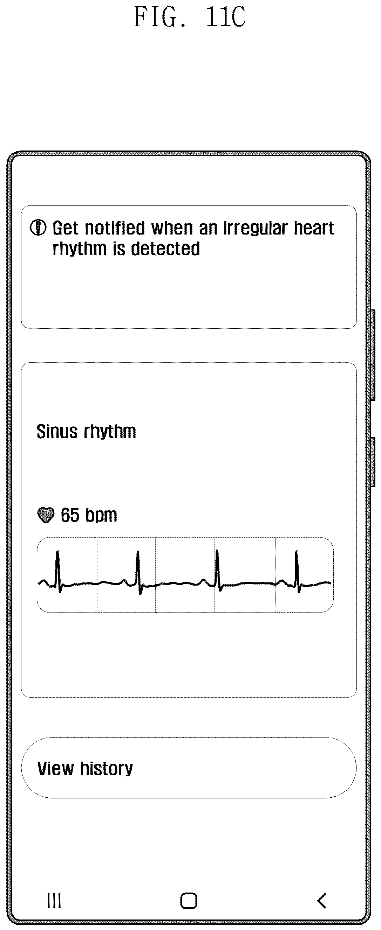

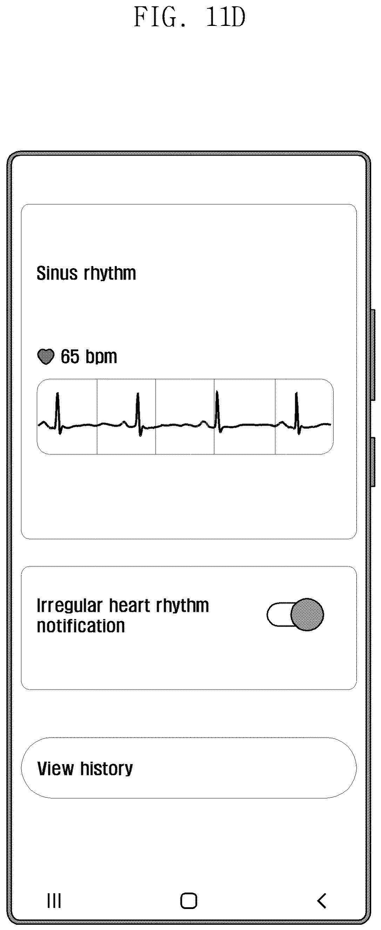

FIGS. 11A, 11B, 11C, and 11D are diagrams illustrating a heartbeat measurement UI according to various embodiments.

Referring to FIG. 11A, an example of a UI displayed when there is no ECG measurement history is illustrated. At this time, the wearable device 200 is unable to perform the PPG measurement function.

Referring to FIG. 11B, an example of a UI displayed when the PPG measurement function is not used and the ECG data is invalid is illustrated. At this time, the wearable device 200 is unable to perform the PPG measurement function.

Referring to FIG. 11C, an example of a UI displayed when the PPG measurement function is not used and the ECG data is valid is illustrated. At this time, the wearable device 200 and/or the electronic device 101 may display a guide for the PPG measurement function.

Referring to FIG. 11D, an example of a UI displayed when the PPG measurement function is used is illustrated. At this time, the wearable device 200 and/or the electronic device 101 may display a guide indicating that the PPG measurement function is running.

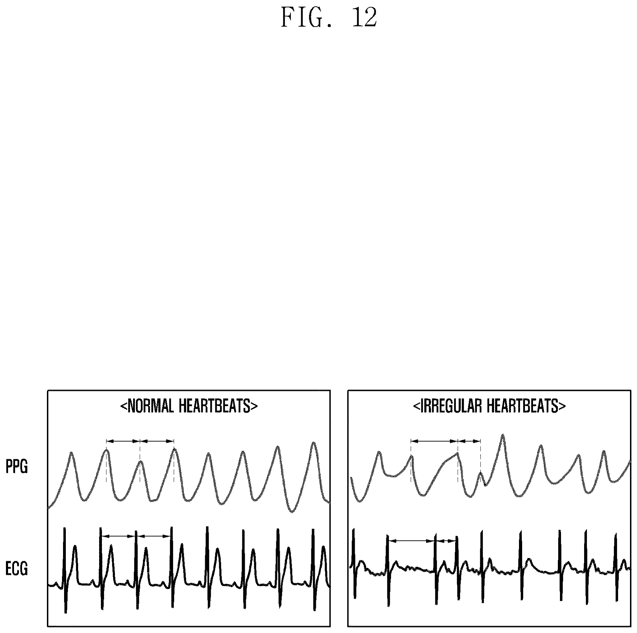

FIG. 12 is a diagram illustrating a heartbeat measurement waveform according to various embodiments.

Referring to FIG. 12, an example of a UI displayed on the wearable device 200 and/or the electronic device 101 when PPG data includes irregular heartbeat information is illustrated. Irregular heartbeats may be estimated when peak information occurs irregularly above an expected value in a biometric signal measured by the PPG sensor 232. PPG may be a signal obtained by optically measuring the amount of blood flow pumped into blood vessels due to heartbeats. Therefore, PPG may generate peak information with a pattern similar to that of ECG, which is a signal obtained by electrically measuring heartbeats, with a slight time lag. When heartbeats are irregular, a pattern of irregularly increasing/decreasing peak intervals may be identified from the PPG data.

However, since peaks may be detected due to signal noise or motion artifacts, the signal may be filtered, and the IHRN determination may be made based on data collected in a state of no movement. For example, arrhythmia (irregular heartbeats) may involve the heart beating unpredictably at arbitrary times. Therefore, the wearable device 200 may further utilize inertial and/or barometric pressure sensor information to determine errors caused by tachycardia that occurs momentarily when sitting down and standing up. For example, even when the wearable device 200 detects irregular heartbeat information from the PPG sensor, it may ignore the irregular heartbeat information in the case where the movement is detected by the inertial sensor or where the barometric pressure information detected by the barometric sensor is high. Alternatively, in this case, the wearable device 200 may measure PPG multiple times to determine whether there is irregular heartbeat information.

The electronic device 101 and/or wearable device 200 may identify abnormal signals (e.g., irregular heartbeat signals) from the ECG data after inducing the ECG measurement and, when an abnormality is determined, connect the user to medical services.

Unlike ECG-based technologies, the PPG-based irregular heartbeat information detection function may significantly contribute to the user's health management by enabling unconscious continuous monitoring. However, due to the measurement principle of the PPG sensor 232, the analysis accuracy may be lower than that of the ECG sensor 231. Therefore, the method and device of this document may supplement accuracy by continuously measuring PPG and, when a possible abnormality is detected, measuring a relatively accurate ECG.

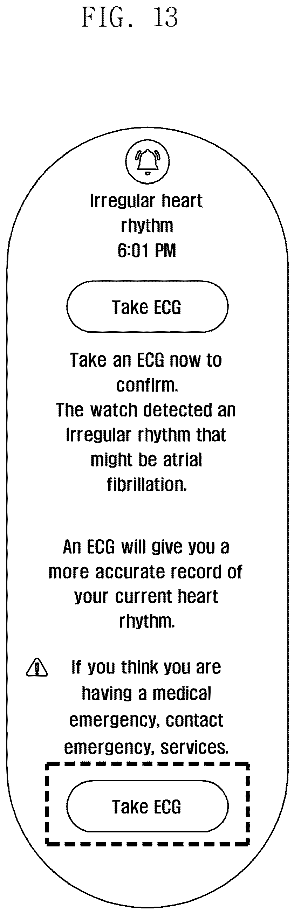

FIG. 13 is a diagram illustrating a UI that provides guidance on ECG measurement according to various embodiments.

Referring to FIG. 13, an example of an alert UI displayed on the wearable device 200 and/or electronic device 101 when irregular heartbeat information is detected in a situation that is not due to noise or motion artifacts is illustrated. When irregular heartbeat information is detected from PPG data, the wearable device 200 and/or the electronic device 101 may display a guide UI for guiding the user to measure ECG for accurate determination.

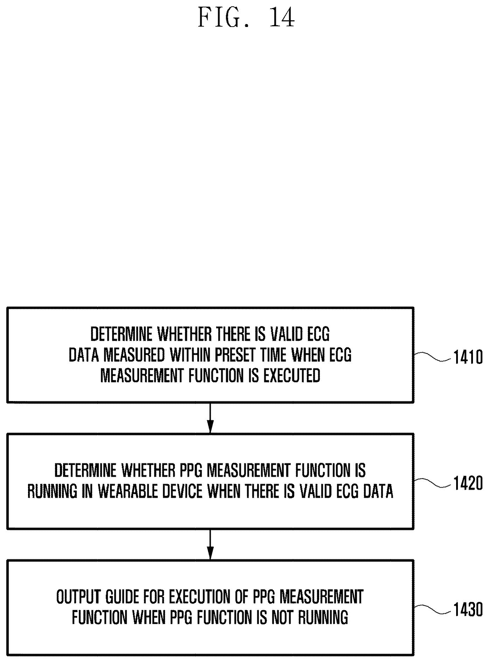

FIG. 14 is a flowchart illustrating a heartbeat measurement method according to various embodiments.

In the following embodiments, respective operations may be performed sequentially, but are not necessarily performed sequentially. For example, the sequence of the respective operations may vary, and at least two operations may be performed in parallel.

According to an embodiment, operations 1410 to 1430 may be understood to be performed by a processor (e.g., the processor 120 in FIG. 2) of an electronic device (e.g., the electronic device 101 in FIG. 2).

When an ECG measurement function is executed, the electronic device 101 may determine whether there is valid ECG data measured within a pre-configured time (1410). For example, the pre-configured time may be set to one week. For example, ECG data and/or ECG analysis results may include sinus rhythm, atrial fibrillation, analysis failure, and/or poor signal quality. When the ECG data and/or ECG analysis results include sinus rhythm, atrial fibrillation, and/or analysis failure, the electronic device 101 may determine the ECG data as valid. Alternatively, when the ECG data and/or ECG analysis results include poor signal quality, the electronic device 101 may determine the ECG data as invalid.

When there is valid ECG data, the electronic device 101 may determine whether a PPG measurement function is running in the wearable device 200 (1420). When the PPG function is not running, the electronic device 101 may output a UI guiding the execution of the PPG measurement function (1430). Alternatively, when the PPG function is running, the electronic device 101 may display a UI indicating that the PPG function is running.

Alternatively, when there is no valid ECG data, the electronic device 101 may output a UI for guiding ECG measurement. For example, when there is no measured ECG data or the signal quality of the measured ECG data is poor, the processor 120 may determine that there is no valid ECG data. When there is no valid ECG data, the electronic device 101 may disable the PPG measurement function. In other words, the electronic device 101 may prompt the user to perform ECG measurement so that valid ECG data may be obtained before PPG measurement. The electronic device 101 may transmit an ECG measurement command to the wearable device 200 according to a user command.

The wearable device 200 may continuously perform the PPG measurement function. When the PPG measurement function is executed in the wearable device 200, the electronic device 101 may receive PPG data from the wearable device at a pre-configured cycle. For example, the electronic device 101 may receive PPG data from the wearable device 200 at regular time intervals. Alternatively, the electronic device 101 may receive PPG data from the wearable device 200 whenever the wearable device 200 performs a measurement.

When the PPG data received from the wearable device 200 includes irregular heartbeat information a pre-configured number of times or more, the electronic device 101 may output a UI for guiding ECG measurement. For example, when the received PPG data includes irregular heartbeat information even once, the electronic device 101 may output a UI for guiding ECG measurement. Alternatively, when the received PPG data includes irregular heartbeat information three or more times, the electronic device 101 may output a UI for guiding ECG measurement. In addition, when the measured ECG data includes irregular heartbeat information, the electronic device 101 may transmit the ECG data (and/or analysis results), the PPG data (and/or analysis results), and/or a medical service request to a server of an external organization (e.g., a hospital). Alternatively, when the received PPG data includes irregular heartbeat information a pre-configured number of times or more, the electronic device 101 may transmit the ECG data (and/or analysis results), the PPG data (and/or analysis results), and/or a medical service request to a server of an external organization (e.g., a hospital).

For example, the heartbeat measurement method may determine whether there is valid ECG data measured within a pre-configured time when the ECG measurement function is executed (1410). When there is valid ECG data, the heartbeat measurement method may determine whether the PPG measurement function is running in the wearable device 200 (1420). The heartbeat measurement method may output a guide for the execution of the PPG measurement function when the PPG function is not running (1430).

For example, when there is no valid ECG data, the heartbeat measurement method may output a guide for ECG measurement and disable the PPG measurement function. The heartbeat measurement method may transmit an ECG measurement command to the wearable device 200 according to a user command.

For example, when the PPG data received from the wearable device 200 includes irregular heartbeat information a pre-configured number of times or more, the heartbeat measurement method may output a guide for ECG measurement.

For example, when ECG measurement data includes irregular heartbeat information, the heartbeat measurement method may transmit at least one of the ECG data, the PPG data, and a medical service request to a server of an external organization.

For example, the heartbeat measurement method may receive PPG data from the wearable device 200 at a pre-configured cycle when the PPG measurement function is executed in the wearable device 200.

For example, the electronic device 101 may include a display 160, a communication interface 190 that performs communication with the wearable device 200, and at least one processor 120. The at least one processor 120 may determine whether there is valid ECG data measured within a pre-configured time when the ECG measurement function is executed. When there is valid ECG data, the at least one processor 120 may determine whether a PPG measurement function is running in the wearable device 200. When the PPG function is not running, the at least one processor 120 may control the display 160 to output a guide for the execution of the PPG measurement function.

For example, when there is no valid ECG data, the at least one processor 120 may control the display 160 to output a guide for ECG measurement. The at least one processor 120 may disable the PPG measurement function. The at least one processor 120 may control the communication interface 190 to transmit an ECG measurement command to the wearable device 200 according to a user command.

For example, when the PPG data received from the wearable device 200 includes irregular heartbeat information a pre-configured number of times or more, the at least one processor 120 may control the display 160 to output a guide for ECG measurement.

For example, when the ECG measurement data includes irregular heartbeat information, the at least one processor 120 may control the communication interface 190 to transmit at least one of the ECG data, the PPG data, and a medical service request to a server of an external organization.

For example, when the PPG measurement function is executed in the wearable device 200, the at least one processor 120 may control the communication interface 190 to receive PPG data from the wearable device 200 at a pre-configured cycle.

It should be appreciated that various examples of the disclosure and the terms used therein are not intended to limit the technological features set forth herein to particular examples and include various changes, equivalents, or replacements for a corresponding example. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it denotes that the element may be coupled with the other element directly (e.g., wiredly), wirelessly, or via a third element.

As used in connection with various examples of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an example, the module may be implemented in a form of an application-specific integrated circuit (ASIC).

Various examples as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an example, a method according to various examples of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various examples, each component (e.g., a module or a program) of the above-described components may include a single entity or multiple entities, and some of the multiple entities may be separately disposed in different components. According to various examples, one or more of the above-described components may be omitted, or one or more other components may be added. Alternatively or additionally, a plurality of components (e.g., modules or programs) may be integrated into a single component. In such a case, according to various examples, the integrated component may still perform one or more functions of each of the plurality of components in the same or similar manner as they are performed by a corresponding one of the plurality of components before the integration. According to various examples, operations performed by the module, the program, or another component may be carried out sequentially, in parallel, repeatedly, or heuristically, or one or more of the operations may be executed in a different order or omitted, or one or more other operations may be added.

The effects obtainable from this document are not limited to the effects mentioned above, and other effects not mentioned will be clearly understood by those skilled in the art from the above description.

## Claims

1. A method for measuring heartbeats, the method comprising:
determining whether there is valid ECG data measured within a pre-configured time in a case where an ECG measurement function is executed;
determining whether a PPG measurement function is running in a wearable device in a case where there is valid ECG data; and
outputting a guide for execution of the PPG measurement function in a case where the PPG function is not running.

2. The method of claim 1, further comprising:
outputting a guide for ECG measurement and disabling the PPG measurement function when there is no valid ECG data; and
transmitting an ECG measurement command to the wearable device according to a user command.

3. The method of claim 1, further comprising:
outputting a guide for ECG measurement in a case where PPG data received from the wearable device comprises irregular heartbeat information a pre-configured number of times or more.

4. The method of claim 3, further comprising:
transmitting at least one of the ECG data, the PPG data, and a medical service request to a server of an external organization in a case where ECG measurement data comprises irregular heartbeat information.

5. The method of claim 1, further comprising:
receiving PPG data from the wearable device at a pre-configured cycle in a case where the PPG measurement function is executed in the wearable device.

6. An electronic device comprising:
a display;
a communication interface configured to communicate with a wearable device; and
at least one processor,
wherein the at least one processor is configured to:
determine whether there is valid ECG data measured within a pre-configured time in a case where an ECG measurement function is executed;
determine whether a PPG measurement function is running in the wearable device in a case where there is valid ECG data; and
control the display to output a guide for execution of the PPG measurement function in a case where the PPG function is not running.

7. The electronic device of claim 6,
wherein the at least one processor is configured to
control the display to output a guide for ECG measurement and disable the PPG measurement function in a case where there is no valid ECG data, and control the communication interface to transmit an ECG measurement command to the wearable device according to a user command.

8. The electronic device of claim 6,
wherein the at least one processor is configured to
control the display to output a guide for ECG measurement in a case where PPG data received from the wearable device comprises irregular heartbeat information a pre-configured number of times or more.

9. The electronic device of claim 8,
wherein the at least one processor is configured to
control the communication interface to transmit at least one of the ECG data, the PPG data, and a medical service request to a server of an external organization in a case where ECG measurement data comprises irregular heartbeat information.

10. The electronic device of claim 6,
wherein the at least one processor is configured to
control the communication interface to receive PPG data from the wearable device at a pre-configured cycle in a case where the PPG measurement function is executed in the wearable device.

11. A non-transitory computer-readable storage medium recording a program for performing a method for measuring heartbeats, the method comprising:
determining whether there is valid ECG data measured within a pre-configured time in a case where an ECG measurement function is executed;
determining whether a PPG measurement function is running in a wearable device in a case where there is valid ECG data; and
outputting a guide for execution of the PPG measurement function in a case where the PPG function is not running.
